(19)

Europäisches Patentamt

European Patent Office

Office européen des brevets

(11) EP 3 925 698 A1

(12) EUROPEAN PATENT APPLICATION

(43) Date of publication:
22.12.2021 Bulletin 2021/51

(21) Application number: 20181096.7

(22) Date of filing: 19.06.2020

(51) Int Cl.:
B01J 13/10 (2006.01)      A01N 25/28 (2006.01)
A61K 8/11 (2006.01)       A61Q 19/00 (2006.01)
B01J 13/14 (2006.01)      B01J 13/22 (2006.01)
C09B 67/02 (2006.01)      C11D 3/50 (2006.01)
F28D 20/02 (2006.01)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(71) Applicant: Follmann GmbH & Co. KG
32423 Minden (DE)

(72) Inventors:
• AMADO MUNOZ, Raul
  32423 Minden (DE)
• REICHEL, Olivier
  32423 Minden (DE)
• MUES, Daniel
  32423 Minden (DE)

(74) Representative: von Renesse, Dorothea et al
König-Szynka-Tilmann-von Renesse
Patentanwälte Partnerschaft mbB
Mönchenwerther Str. 11
40545 Düsseldorf (DE)

(54) IMPROVED MICROCAPSULES AND METHOD FOR THE PRODUCTION AND USE THEREOF

(57) The present invention is directed to a method for producing environmentally friendly microcapsules. Moreover, the present invention provides microcapsules obtained/obtainable by the method according to the invention, in particular multilayer microcapsules which are suitable for all kinds of industrial application, in particular in personal care and home care products. The microcapsules preferably comprise at least one oil as core material.

EP 3 925 698 A1

**Description**

The present invention pertains to a method for producing microcapsules, in particular biodegradable core-shell microcapsules comprising core material and shell material, microcapsules produced therewith as well as their uses and compositions comprising them.

Microcapsules allow the manufacture of products and applications with added value in all parts of industries including healthcare and personal care products, agriculture, material science and construction. In homecare products, for example laundry products, microcapsules are frequently used for achieving long lasting freshness, extending the scent of perfume oil or controlled release of the encapsulated substances (core material). Various mechanisms for a controlled release of the core material exist, for example mechanical stress, change of temperature or chemical impacts (pH shift, UV etc.). Many core-shell microcapsules, in particular, microcapsules with high stability have shells containing synthetic resins, such as melamine-formaldehyde resins, urea-formaldehyde resins or phenol-formaldehyde resins.

Microcapsules based on synthetic resins contribute to the environmental burden of microplastic. Solid phase materials are considered as microplastic if the particles are not soluble in water and consist of plastics with a particulate size of 5 millimetres or smaller. Microplastic particles endanger the ecosystem on a long-term basis, including impacts on human health.

It is thus an objective of the present invention to provide a process for the manufacture of environmentally friendly microcapsules. In particular such microcapsules should have a high quality in particular in terms of stability and impermeability in order to fulfill the needs of a long lasting and controlled release of the core material comprised therein.

**SUMMARY OF THE INVENTION**

This problem is solved by a method according to claim 1. Particularly advantageous embodiments of the invention are subject matter of depended or further independent claims.

The method according to the invention is generally based on the known concept of microencapsulation by coacervation of at least two polyelectrolytes, for of example chitosan and gum arabic. However, the known methods are either not suitable for industrial production and/or do not lead to a satisfactory quality or of the resulting microcapsules (e.g. WO2018/183150 A1, see comparative example below - Example 3).

The inventors have found that starting the process of encapsulation with at least two polyelectrolytes being solubilized in the same composition in one step (step a), then emulsifying/suspending a core material in this solution in a further step (in step b), followed by a step of changing the pH value into a pH range wherein the coacervation occurs (step c), leads to microcapsules of high quality and of high yield (encapsulation efficiency and/or dry yield of produced microcapsules). Preferably in step c) the pH value is changed by in- or decreasing the pH value into a pH range wherein the coacervation occurs with the provisio that for the combination of gum arabic and chitosan as polyelectrolytes a pH decrease is excluded. The invention enables an effective industrial production of microcapsules, which are satisfactory in particular in terms of stability and impermeability. These capsules do not require the use of synthetic resin, and therewith fulfill the need for more environmentally friendly microcapsules for industrial needs. Environment-friendly (eco-friendly) microcapsules are in particularly those which are biodegradable and/or comprising biopolymers and/or contain a low amount or even no toxic or hazardous substances. Preferred microcapsules of the invention do not contribute substantially to the microplastic contamination.

Microcapsules obtained/obtainable by the method of the invention exhibit excellent resistance against evaporation of the core material when the capsules are in the dry state as well as excellent resistance against destabilization of the capsules even in harsh environments, e.g. in detergent or surfactant solutions (see below, example 4). Moreover the release profiles can be adjusted by the used shell material(s) - their compositions and thickness.

Furthermore, small microcapsules with a size of d(90%) <50 $\mu$m (measured in deionized water at room temperature by DLS) are obtained/obtainable according to one embodiment of the invention. It is preferred, that the mixing intensity, especially by stirring, is adjusted in the step of emulsifying/suspending a core material (in step b). More preferred is that the mixing intensity, e.g. stirring speed is increased. The mixing intensity, e.g. speed of stirring can be increased to a point where the formed emulsion has a droplet size which is desired to be encapsulated by a coacervate. A skilled person in the art is aware of the fact that the needed mixing intensity is also depending from the reaction vessel/container (volume and geometry). Stirring speeds (in rpm) mentioned herein are always based on a 1-liter batch size. For larger batches, the mixing intensities, e.g. stirring speeds are adjusted according to the used vessel volume and/or geometry. It is further preferred that the mixing intensity, e.g. stirring speed is reduced after the emulsification/suspending in step b) and before raising the pH in step c) before the coacervation step. This enables also capsules of a size of below 25 $\mu$m d(90%).

The microcapsules (abbreviated to capsules) according to the invention comprise a core material. The chemical nature and structure thereof are not specifically limited. It can be a single substance or a mixture of substances. A hydrophobic substance or a mixture of substances comprising at least one hydrophobic substance is preferred.

The core material can comprise a microcapsule (hereinafter also called "template microcapsule"). In this preferred embodiment of the invention a multilayer microcapsule is formed. The method of production of such multilayer microcapsules is a two-step process, comprising in a first step the formation or provision of the template microcapsule and then, in a second step, using the template microcapsule as the core material (or parts thereof) in the process according to the invention, which leads to the production of the outer shell of the multilayer microcapsule.

Advantageously the template microcapsule is a core-shell microcapsule. In one preferred embodiment the shell of the template microcapsule (also called hereinafter the "inner shell" of the multilayer microcapsule) comprises a synthetic resin. Various methods and resin forming polymers and prepolymers for the production of such microcapsules are known to the skilled person. With an inner shell comprising a synthetic resin the impermeability of the multilayer microcapsules can be increased. Hence, such multi-layer microcapsules are particularly advantageous in terms of high impermeability (conferred by the inner shell) and high mechanical stability (conferred by the outer shell). In view of the mechanical stability conferred by the outer shell, the thickness of the inner shell can be reduced. Preferably the amount of used monomers for producing the inner shell are reduced, more preferred are reduced by at least 70 %. Therewith the overall content of the synthetic resin of such multilayer microcapsule is limited, which substantially reduces their environmental impact.

In one embodiment of the invention, the core material can comprise solid particle/s. According to a method of the invention it is therefore possible to encapsulate solid particle/s. The solid particles themselves have no core-shell structure. The solid particle/s can be encapsulated in addition to further core materials. They can comprise a resin, a biopolymer and/or a zeolite. The material of the solid particles is not limited. Preferably the solid particles are porous.

It was also found by the inventors that the use of gum arabic and chitosan as polyelectrolytes are particularly suitable for the production of biodegradable microcapsules.

Other objects, features, advantages and aspects of the present application will become apparent to those skilled in the art from the following description and appended claims. It should be understood, however, that the following description, appended claims, and specific examples, while indicating preferred embodiments of the application, are given by way of illustration only.

**BRIEF DESCRIPTION OF THE FIGURES**

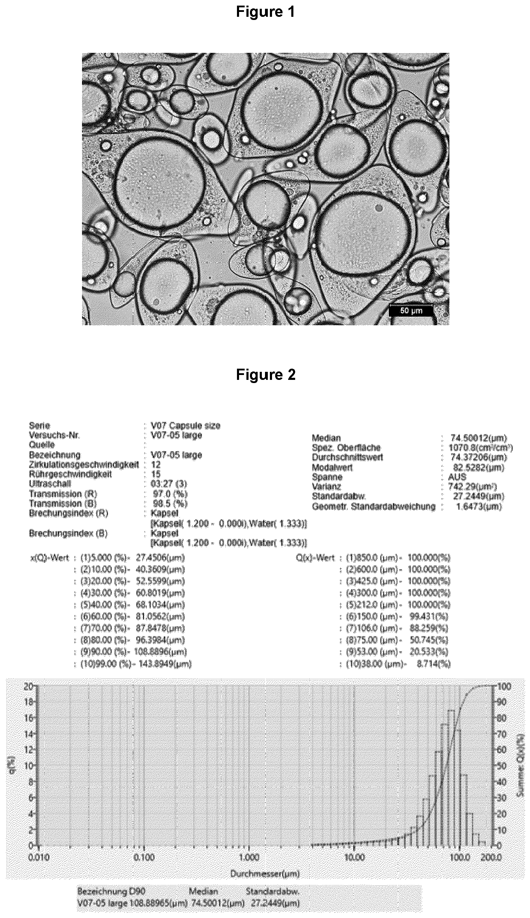

**Fig. 1** is a microscopic image of microcapsules produced by a method according to the invention; shown here are microcapsules wherein chitosan with a degree of deacetylation of >92.7 % was used and the stirring speed in step b) was 600 rpm.

**Fig. 2** is an overview of a DLS measurement and setup from microcapsules shown in Fig. 1.

**Fig. 3** is a microscopic image of microcapsules produced by a method according to the invention; shown here are microcapsules wherein chitosan with a degree of deacetylation of >92.7 % was used and the stirring speed in step b) was 1000 rpm.

**Fig. 4** is an overview of a DLS measurement and setup from microcapsules shown in Fig. 3.

**Fig. 5** is a microscopic image of microcapsules produced by a method according to the invention; shown here are microcapsules wherein chitosan with a degree of deacetylation of >92.7 % was used and the stirring speed in step b) was 3000 rpm.

**Fig. 6** is an overview of a DLS measurement and setup from microcapsules shown in Fig. 5.

**Fig. 7** is a microscopic image of microcapsules produced by a method according to the invention; shown here are microcapsules wherein chitosan with a degree of deacetylation of >70 % was used and the stirring speed in step b) was 1000 rpm.

**Fig. 8** is an overview of a DLS measurement and setup from microcapsules shown in Fig. 7.

**Fig. 9** is a microscopic image of microcapsules produced by a method according to the invention; shown here are microcapsules wherein chitosan with a degree of deacetylation of >70 % was used and the stirring speed in step b) was 3000 rpm.

**Fig. 10** is an overview of a DLS measurement and setup from microcapsules shown in Fig. 9.

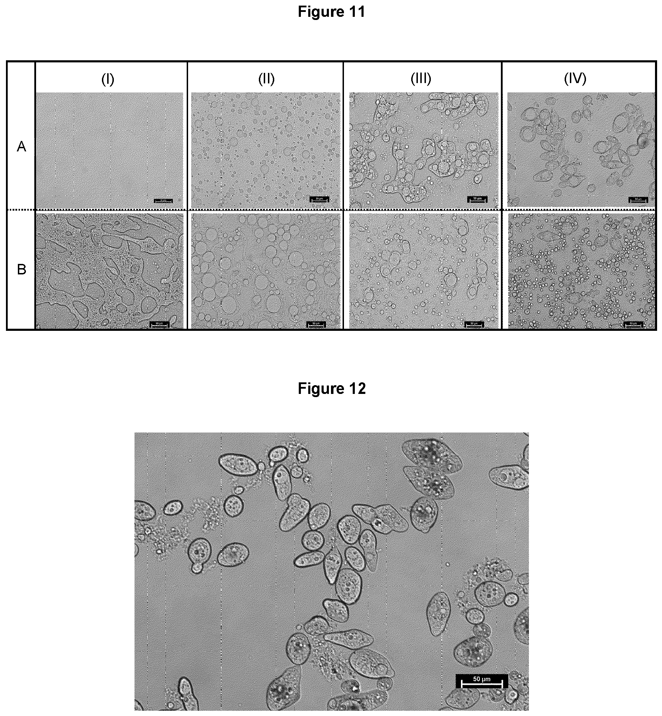

**Fig. 11** is a contrasting juxtaposition over four steps of production of microcapsules with a coacervate of chitosan and gum arabic as shell material; step (I) solution of the two polyelectrolytes at the starting pH, step (II) creation of an emulsion by stirring, step (III) changing the pH into the pH range of coacervate (here pH 4.1) by in- or decreasing the pH, step (IV) crosslinking - *top raw:* method according to the invention (starting point at a pH value which is below (<) the pH range of the coacervation of the used polyelectrolytes) versus *bottom raw:* a state of the art method (starting point at a pH value which is above (>) the pH range of the coacervation of the used polyelectrolytes).

**Fig. 12** is a microscopic image of the slurry of encapsulated Coumarin 1-containing octane under UV light according to a method according to the invention.

**Fig. 13** is a microscopic image of the slurry of encapsulated Coumarin 1-containing octane under UV light according to a state of the art method.

**Fig. 14** is a graphic showing the results of a thermal analysis of microcapsules from the comparative test showing core material loss as a function of temperature for "Cap Octan pH 2.5" and "Cap Octan pH 4.7.

**Fig. 15** is a microscopic image of microcapsules produced by a method according to the invention; shown here are microcapsules with a shell of a coacervate of chitosan and gum arabic and a core material which is a second microcapsule / template microcapsule encapsulating fragrance oil with a shell of aminoplastic resin.

**Fig. 16** is a microscopic image of a microcapsule produced by a method according to the invention; shown here is a microcapsule with a shell of a coacervate of chitosan and gum arabic and a core material which is a second microcapsule / template microcapsule encapsulating fragrance oil with a shell of phenoplastic (phenolic) resin.

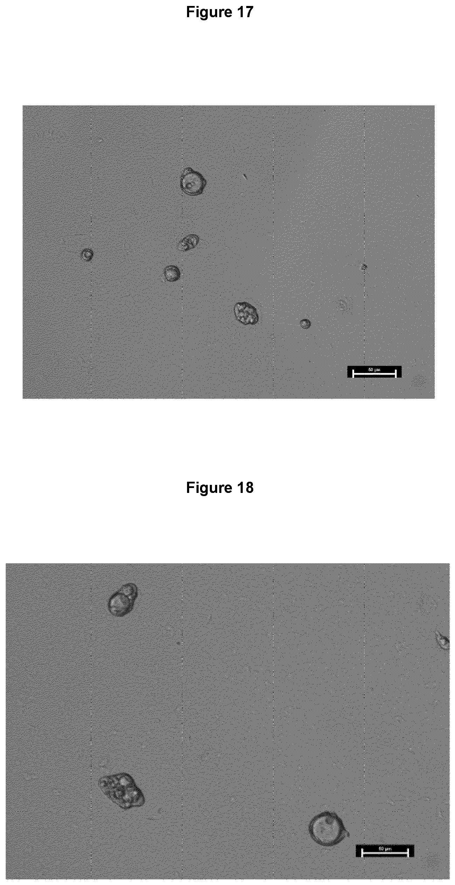

**Fig. 17** is a microscopic image of multilayer microcapsules (R14-03-09) produced by a method according to the invention; shown here are microcapsules with a shell of a coacervate of chitosan and gum arabic and a core material which is a second microcapsule / template microcapsule encapsulating fragrance oil coloured by the addition of Courmarin 1 with a thin shell of phenolic resin (90 % reduced synthetic polymer in comparison to commercially available capsules for laundry, see table 1). These shown multilayer microcapsules were stored in a standard softener (purchased from Henkel, pH = 3.0) for 14 days at 45 °C. The image shows no loss/leakage of core material. The multilayer microcapsules are still stable and leak-proof, even after harsh storage conditions.

**Fig. 18** is a microscopic image of multilayer microcapsules (R14-03-14) produced by a method according to the invention; shown here are microcapsules with a shell of a coacervate of chitosan and gum arabic and a core material which is a second microcapsule / template microcapsule encapsulating fragrance oil coloured by the addition of Courmarin 1 with a thin shell of phenolic resin (80 % reduced synthetic polymer in comparison to commercially available capsules for laundry, see table 1). These shown multilayer microcapsules were stored in a standard softener (purchased from Henkel, pH = 3.0) for 14 days at 45 °C. The image shows no loss/leakage of core material. The multilayer microcapsules are stable and leak-proof, even after harsh storage conditions.

**Fig. 19** are bar chart results from the sensory panel at day 0 and after 14 days of storage at 45 °C (before and after rubbing) of two different multilayer capsules according to the invention.

## DETAILED DESCRIPTION OF THE INVENTION

The present invention provides an improved method for producing environmentally friendly microcapsules. Moreover, the present invention provides microcapsules obtainable by the method according to the invention, in particular multilayer microcapsules which are suitable for all kinds of industrial application, in particular in personal care and home care products. The microcapsule preferably comprises at least one oil as core material.

In one embodiment of the invention a method is disclosed for producing microcapsules comprising core material and shell material comprising the following steps,

a) providing a composition comprising at least two solubilized polyelectrolytes,
b) adding a core material to the composition of step a) and
c) initiating the coacervation by changing the pH value into the pH range wherein coacervation of the polyelectrolytes occurs.

In an preferred embodiment of the invention in step c) the coacervation is initiated by in- or decreasing the pH value into the pH range wherein coacervation of the polyelectrolytes occurs with the provisio that for the combination of gum arabic and chitosan as polyelectrolytes a pH decrease is excluded. In a more preferred embodiment of the invention in step c) the coacervation is initiated by increasing the pH value into the pH range wherein coacervation of the polyelectrolytes occurs.

"Polyelectrolytes" according to the invention are polymers whose repeating units bear an electrolyte group. Polycations and polyanions are polyelectrolytes. These groups dissociate in aqueous solutions (water), making the polymers charged. Polyelectrolyte properties are thus similar to both electrolytes (salts) and polymers (high molecular weight compounds) and are sometimes called polysalts. Like salts, their solutions are electrically conductive. Like polymers, their solutions are often viscous. They can be classified as cationic, anionic, and nonionic according to the nature of the functional groups along the polymer chain. Polyelectrolytes can be of natural or synthetic origin. Depending on their nature or composition, biopolymers can also be polyelectrolytes.

"Coacervation" (shall also include "complex coacervation") is a phenomenon that produces coacervate colloidal droplets. When coacervation occurs, two liquid phases will co-exist: a dense, polymer-rich phase (coacervate phase or coacervate droplets) and a dilute, polymer-deficient phase (dilute phase). It is caused when the solubility of at least two oppositely charged macromolecular substance in a solvent is reduced by appropriate measures, e.g. by the change of pH. One example is the coacervation of the polyelectrolytes gum arabic and chitosan. The coacervation takes place as a result of interactions between the carboxyl anion groups of the gum arabic ($-COO^-$) and the protonated amino groups of the chitosan ($-NH_3^+$). As a result of these interactions, a complex with reduced solvent (e.g. water) solubility is formed which deposits at the phase boundary of the emulsified droplets of the hydrophobic phase (e.g. oil droplets). Therewith a core-shell microcapsule is formed.

The shell material/s of the microcapsules according to the invention can be selected from natural, semi-synthetic or synthetic materials or a mixture thereof.

Natural shell materials are inorganic materials like zeolites or carbonates like calcium carbonate or organic materials like gum arabic, agar-agar, agarose, maltodextrins, alginic acid or its salts, e.g. sodium or calcium alginate, fats and fatty acids, cetyl alcohol, collagen, chitosan, lecithin, gelatin, albumin, shellac, polysaccharides such as starch or dextran, polypeptides, protein hydrolysates, sucrose and waxes.

Semi-synthetic shell materials are chemically modified celluloses, especially cellulose esters and ethers of cellulose, e.g. cellulose acetate, ethyl cellulose, hydroxypropyl cellulose, hydroxypropyl methyl cellulose and carboxymethyl cellulose, as well as starch derivatives, especially starch ethers and esters.

Synthetic shell materials are polymers such as polyacrylates, polyamides, polyvinyl alcohol, polyuria, polyurethane or polyvinyl pyrrolidone or (thermosetting) resins like phenoplastic and/or aminoplastic resins and/or modified silica.

In a particular preferred embodiment the shell material of the microcapsules comprise one or more biopolymers, more preferred consist of biopolymers.

The term "biopolymers" describes polymers which are obtainable in nature; in other words, they are polymeric biomolecules derivable from cellular or extracellular matter. Biopolymers contain monomeric units that are covalently bonded to form larger structures. Biopolymers can be classified into three main classes, classified according to the monomeric units used and the structure of the biopolymer formed: polynucleotides, polypeptides, and polysaccharides. Common biopolymers, just to name a few, are for example collagen, silk fibroin, gelatin, starch, cellulose, alginate, gum arabic and chitosan.

In a preferred embodiment the method according to the invention comprises a step of crosslinking (step d). The crosslinking is accomplished e.g. by adding a covalent crosslinker such as an aldehydic compounds, e.g. glyoxal, formaldehyde, glutaraldehyde, genipin or aglycone geniposidic acid, vanillin, ethylene glycol diglycidyl ether (EGDE), hexamethylene-1,6-di-(aminocarboxysulfonate) or mixtures thereof. Alternatively ionic crosslinkers such as tannic acid or tripolyphosphates, e.g. sodium tripolyphosphate (NaTPP), citric acid or mixtures thereof such as a mixture of glutaraldehyde and tannic acid, are possible. A particular preferred crosslinker is glutaraldehyde.

According to the invention the composition of step a) has a pH value which is below or above the pH range of the coacervation of the used polyelectrolytes with the provisio that a pH value above the pH range of the coacervation for a composition comprising a combination of gum arabic and chitosan as polyelectrolytes is excluded.

In a preferred embodiment, the composition of step a) has a pH value which is below the pH range of the coacervation of the used polyelectrolytes.

In a more preferred embodiment, the composition of step a) has a pH value, that is greater than or equal ($\geq$) to the p$K_a$ value of one of the polyelectrolytes and below (<) of the pH range of coacervation of the used polyelectrolytes.

In general, a skilled person in the art knows how to determine the pH range of the coacervation, which depends on the used polyelectrolytes, their quantities and proportions, as well as the reaction temperature. These specific pH ranges can be determined for example in turbidity studies.

In a particular preferred embodiment of the method according the invention in step a) the polyelectrolytes are dissolved in a solution at an acidic pH, preferably at a pH value below (<) 2.5. In step b) they are then dispersed or suspended with the core material. In step c) the pH is increased to a pH range wherein coacervation of the polyelectrolytes occurs, preferably to a pH value above (>) 2.5. This embodiment of the invention is particularly suitable when gum arabic and chitosan are applied.

The compositions used in step a) of the present invention in general can be of any type or nature, for example a one phase or two phase system, such as an emulsions or dispersions, as long as the at least two polyelectrolytes are solubilized therein.

In a particular preferred embodiment the polyelectrolytes include at least one cationic polyelectrolyte and one anionic polyelectrolyte.

Preferred polyelectrolytes are selected from the group consisting of gum arabic, sodium carboxymethyl guar gum, plant gums, chitosan, gelatin, casein, pectin, plant proteins, egg white protein, silk protein, alginate, chitin, glycogen, cellulose (in particular carboxymethyl cellulose), starch (e.g. amylose or amylopectin), hyaluronic acid, poly(styrene sulfonate), lignin sulfonate or xanthan gum or mixtures thereof, preferably gum arabic and chitosan or gum arabic and gelatin or gum arabic and soy protein isolate or casein and pectin. Most preferred is the use of gum arabic and chitosan.

Chitosan is a linear polysaccharide (and polyelectrolyte) composed of randomly distributed $\beta$-(1→4)-linked D-glucosamine (deacetylated unit, GlcN) and N-acetyl-D-glucosamine (acetylated unit, GlcNAc). Chitosan is produced commercially by deacetylation of chitin. Chitosan may have different degrees of deacetylation. Due to the free amino groups formed by deacetylation, it is a polycation with a high charge density in non-alkaline solution. The ratio of both monomers (Glcn and GlcNAc) to each other is described by the degree of deacetylation $D_{deac}$. If there is no explicit indication of the degree of deacetylation, "chitosan" of any degree of deacetylation is understood.

Chitosan, preferably being used in the present invention has an average molecular weight comprised from 10 to 90 kDa, more preferably from 15 to 80 kDa.

Chitosan being used may have a degree deacetylation of greater than or equal to 70 by mol%, relative to the wet weight.

In one embodiment the used chitosan has a degree of deacetylation greater 70 mol%, preferred of greater than or equal to 95 mol%.

In one embodiment the used chitosan has a degree of deacetylation of above 70 mol% and a molecular weight from 15 to 80 kDa.

In another embodiment of the method according the invention additives may be added after the coacervation step c) to avoid agglomeration effects. Useful additives are natural anionic polymers such as carboxylmethyl cellulose or anionic polysaccharides such as carrageenan, pectin, xanthan, gellan or lignin sulfonates or synthetic (co)polymers such as polyacrylic acids, polymethacrylic acids, polystyrene sulfonic acids or polyphosphonic acids.

It is also possible to add thickener during the coacervation step such as hydroxypropylmethyl cellulose or derivatives thereof, modified polysaccharides, e.g. hydroxypropyl guar or synthetic (co-)polymers, e.g. polyvinyl alcohols, polyacrylamides, polyethylene glycol acrylates or -methacrylates and polyethylene glycol.

Steps b) and c) of the method according to the invention are preferably performed under stirring. In a preferred embodiment the stirring speed in step b) is adjusted between 600 and 3000 rpm, preferably between 1000 and 2000 rpm and is reduced to 300 to 500 rpm shortly before the coacervation in step c) occurs. For step c) it is preferred to have a stirring speed between 300 to 500 rpm. The indicated stirring speeds herein refer to a 1 liter batch e.g. in a common laboratory dissolver. The person skilled in the art knows how to adjust the stirring speed according to the reaction vessel used (e.g. larger reaction vessel requires adapted stirring speeds).

In a particular preferred embodiment of the invention, the core material of the microcapsules comprises at least one hydrophobic substance or a mixture of various substances containing one or more hydrophobic substance. The hydrophobic substance is preferably selected from the group comprising alcohols, perfume oils, care substances, natural and/or synthetic oils, silicone oils, pesticides, biocides, pigments, phase change materials (PCM), fertilizer, adhesives, insecticides, solvents, lubricants, dyes and cooling substances or mixtures thereof. The core : shell ratio is preferably in the range between 1:1 and 2:1.

In a preferred embodiment of the present invention the core material comprises one or more microcapsule/s (hereinafter also called "template microcapsule/s"). This preferably is a core-shell microcapsule, comprising e.g. at least one hydrophobic ingredient. Therewith the method of the invention provides multi-layer microcapsules. The inner shell preferably contains a resin.

The template microcapsule may be produced (version A) by a condensation of thermosetting resins and a subsequent formation of microcapsules using aldehydes, preferably formaldehyde. A process for the preparation of such microcapsules is disclosed e.g. in WO 2009/015872 A1, which is expressly incorporated herein by reference.

In a preferred embodiment of the invention, the template microcapsule is produced (version B) by reacting

a) at least one aromatic alcohol or ethers or esters thereof and
b) at least one aldehyde component that comprises at least two carbon atoms per molecule, and

c) in the presence of at least one homopolymer or copolymer of 2-acrylamido-2-methyl-propane sulfonic acid or salts thereof.

Suitable (in version B) as an aromatic alcohol a) are compounds selected from phenol, cresol (o-, m- and p-cresol), naphthol ($\alpha$- and $\beta$-naphthol), thymol, pyrocatechol, resorcinol, hydroquinone and 1,4-naphthohydroquinone, phloroglucinol, pyrogallol, or hydroxyhydroquinone.

Suitable (in version B) as an aldehydic component b) are compounds selected from valeraldehyde, capronaldehyde, caprylaldehyde, decanal, succindialdehyde, cyclohexanecarboxaldehyde, cyclopentanecarboxaldehyde, 2-methyl-1-propanal, 2-methylpropionaldehyde, acetaldehyde, acrolein, aldosterone, antimycin A, 8'-apo-$\beta$-carotene-8'-al, benzaldehyde, butanal, chloral, citral, citronellal, crotonaldehyde, dimethylaminobenzaldehyde, folinic acid, fosmidomycin, furfural, glutaraldehyde, glyceraldehyde, glycolaldehyde, glyoxal, glyoxylic acid, heptanal, 2-hydroxybenzaldehyde, 3-hydroxybutanal, hydroxymethylfurfural, 4-hydroxynonenal, isobutanal, isobutyraldehyde, methacrolein, 2-methylundecanal, mucochloric acid, N-methylformamide, 2-nitrobenzaldehyde, nonanal, octanal, oleocanthal, orlistat, pentanal, phenylethanal, phycocyanin, piperonal, propanal, propenal, protocatechuic aldehyde, retinal, salicylaldehyde, secologanin, streptomycin, strophanthidin, tylosin, vanillin, or cinnamaldehyde.

In a preferred embodiment (in version B) the molar ratio of the aromatic alcohol a) to the aldehydic component b), which comprises at least two carbon atoms per molecule, is between 1:2 and 1:3.5, preferably between 1:2.4 and 1:2.8, and is particularly preferably between 1:2.6.

This process (Version B) which is suitable for the manufacture of template microcapsules is in detail described in WO 2011/110368 A1, and consequently these documents are incorporated in their totality into the disclosure content of the present invention by way of reference.

In a further embodiment of the method according to the invention at least one surfactant may be used during the production of the template microcapsules. Preferably, the surfactant is selected from the group consisting of sulfonated lignin, natural dispersants like quillaja saponin, lecithins, gum arabic, pectin, carrageenan, chitosan, chondroitin sulfate, cellulose gum, physically or chemically modified starch, whey protein, pea protein, egg white protein, silk protein, gelatin of fish, proteins of porcine or bovine origin, ester gum, fatty acids or mixtures thereof, sulfonated lignin is preferred.

Suitable as protective colloids in the production of microcapsules are AMPS-copolymers described in detail in WO 2010/102830 A1 which is incorporated into the disclosure content of the present invention by way of reference. Examples of AMPS-copolymers are AMPS/hydroxyethylmethacrylate-copolymer, AMPS/hydroxyethylacrylate-copolymer, AMPS/hydroxypropylmethacrylate-copolymer, AMPS/hydroxypropylacrylate-copolymer, AMPS/hydroxybuylmethacrylate-copolymer, AMPS/hydroxybutylacrylate-copolymer, AMPS/polyethylene glycol monomethacrylate-copolymer, AMPS/polypropylene glycol monomethacrylate-copolymer, AMPS/polypropylene glycol monoacrylate-copolymer, AMPS/methoxy-polyethylene glycol monomethacrylate-copolymer, AMPS/methoxypolyethylene glycol monoacrylate-copolymer, AMPS/hydroxyethylmethacrylate-copolymer, AMPS/hydroxyethylacrylate-copolymer, AMPS/hydroxypropylmethacrylate-copolymer, AMPS/hydroxypropylacrylate-copolymer, AMPS/hydroxybutylmethacrylate-copolymer, AMPS/hydroxybutylacrylate-copolymer, AMPS/polyethylene glycol monomethacrylate-copolymer, AMPS/polypropylene glycol monomethacrylate-copolymer, AMPS/polypropylene glycol monoacrylate-copolymer, AMPS/methoxy-polyethylene glycol monomethacrylate-copolymer, AMPS/methoxy-polyethylene glycol monoacrylate-copolymer.

In a further embodiment of the method according to the invention the core material in step b) comprises at least one hydrophobic ingredient which is selected from the group consisting of alcohols, perfume oils, care substances, natural and/or synthetic oils, silicone oils, pesticides, biocides, pigments, phase change materials (PCM), fertilizer, adhesives, insecticides, solvents, lubricants, dyes and cooling substances or mixtures thereof.

Particularly a microcapsule according to the invention is most preferred, wherein the inner shell material (the shell material of the template microcapsule) comprises a synthetic polymer, especially preferred an aminoplastic and/or phenoplastic resin and the shell material (the outer shell material of the microcapsules) comprises at least one biopolymer, especially preferred comprises gum arabic and chitosan.

In a preferred embodiment, microcapsules produced by a method according to the invention have a low content of synthetic polymer(s).

The "content of synthetic polymer(s)" (abbreviated to: CSP) is the ratio of synthetic polymer(s) to encapsulated core material. It describes the amount of synthetic polymer (e.g. in gram, g) used/needed to encapsulate a certain amount of core material (e.g. in gram, g) - the quotient of synthetic polymer (abbreviated to: SP) to core material (abbreviated to: CM).

As an example, commercially available phenolic resin microcapsules for laundry need at least 30 g synthetic polymer(s) (SP) to encapsulate 100 g of core material (CM) (to deliver stable microcapsules, which are stable even in harsh conditions like softener or detergents); CSP = 0.3. In one method according to the invention the produced multilayer microcapsules need less than 15 g synthetic polymer(s) (SP) per 100 g core material (CM), preferably less than 10 g synthetic polymer(s) per 100 g core material; CSP = 0.1.

As a consequence, producing microcapsules by a method according to the invention reduces the content of synthetic polymer(s) by 30 %, preferred by 60 %, more preferred by 75 %, most preferred by 80 %.

Microcapsules according to one preferred embodiment have a content of synthetic polymer(s) (CSP) less than or equal to ($\leq$) 0.15, wherein the CSP is the quotient of the amount of synthetic polymer(s) encapsulating an amount of core material.

In a more preferred embodiment the multilayer microcapsules have a CSP of less than or equal to ($\leq$) 0.15, preferred less than (<) 0.1, more preferred less than (<) 0.08.

The value for CSP can be determined by any method known by a skilled person in the art. Preferably the value for CSP can be determined in a first step, wherein the amount of synthetic polymer (SP) is determined by subtracting the amount of not reacted / unreacted monomers or other educts that were used to create (inner) shell material comprising synthetic polymer(s) during the production process of the microcapsules from the amount of monomers or other educts that were originally used to create (inner) shell material comprising synthetic polymer(s) during the production process of the microcapsules. In a second step, the amount of core material (CM) is determined by subtracting the amount of free / not encapsulated core material (core material which is not encapsulated during the production process of the microcapsules) from the amount of core material originally used to be encapsulated as core material during the production process. The quotient of SP and CM delivers the value for CSP.

The temperature of step a) according to the invention is between 20 °C and 45 °C, preferably between 20 °C and 40 °C, more preferred at 25 °C.

According to a further embodiment, the temperature is kept at 25 °C in steps a) to c).

In another embodiment is the temperature in step d) preferably between 40 °C and 80 °C, more preferred between 45 °C and 50 °C, most preferred at 50 °C.

In one embodiment the method according to the invention may comprise a further step e) wherein the slurry obtained in step d) is dried to provide dried microcapsules.

Typically, the microcapsules according to the invention have a hydrodynamic diameter d(90%) of between 1 $\mu$m and 5.000 $\mu$m, preferably between 5 $\mu$m and 1.000 $\mu$m, more preferably between 15 and 150 $\mu$m, even more preferably between 30 and 120 $\mu$m, most preferred between 40 and 100 $\mu$m, with a standard deviation of $\pm$ 30 $\mu$m (measured via DLS in deionized water at room temperature, volume-weighted evaluation).

The size of the microcapsules according to the invention could be adjusted by the mixing intensity of the emulsion or dispersion, especially by stirring (depending on the rounds per minute, rpm). Furthermore varying the viscosity of dispersed and/or continuous phase may be suitable to adjust the size of the microcapsules.

Furthermore, the microcapsules size may be influenced by the nature of the polyelectrolyte. For example, chitosan with a degree of deacetylation of >70 % leads to small microcapsules with a d(90%) of below 50 $\mu$m.

The indication of microcapsules sizes (= particle diameter or microcapsules diameter) under the suffix "d(90%)" describes the microcapsules size at which 90 % of the measured microcapsules are distributed. For example, d(90%) = 50 $\mu$m means, that 90 % of the microcapsules in the sample measured have a size of 50 $\mu$m.

In one embodiment of the method according to the invention the speed of stirring is adjusted to 600 rpm, which results in microcapsules with a size of d(90%) = 110 $\mu$m $\pm$ 30 $\mu$m.

In a further embodiment of the method according to the invention the speed of stirring is adjusted to 1000 rpm, which results in microcapsules with a size of d(90%) = 60 $\mu$m $\pm$ 30 $\mu$m.

In just another embodiment of the method according to the invention the speed of stirring is adjusted to 3000 rpm, which leads to microcapsules with a size of d(90%) = 50 $\mu$m $\pm$ 30 $\mu$m.

In preferred embodiment microcapsules with a size of d(90%) = 20 $\mu$m $\pm$ 10 $\mu$m are obtained by adjusting the speed of stirring to 3000 rpm (related to a batch size of 1 liter) and by using chitosan as one electrolyte with a degree of deacetylation of >70 %.

In a further preferred embodiment microcapsules with a size of d(90%) = 50 $\mu$m $\pm$ 15 $\mu$m are obtained by using chitosan with a degree of deacetylation of >70 %.

Furthermore, in one embodiment of the method according to the invention, the ratio of the first polyelectrolyte and the second polyelectrolyte in step a) is between 1:1 and 2:1, more preferred about 1:3 and 1:5.

In a preferred embodiment of the method according to the invention, the ratio of chitosan to gum arabic in step a) is preferred between 1:3 and 1:5, more preferred about 1:4.

The ratio of core material to shell material in step a) of the method according to the invention is preferably between 1:10 and 10:1, more preferred between 1:4 and 4:1, most preferred between 2:3 and 3:2.

The microcapsules according to the invention are stable in storage after drying for at least 14 days, preferably for at least 28 days, particularly preferably for at least 36 days at storage temperatures from 20 to 45 °C and at a relative air humidity of 30 to 70 %.

The advantageous properties of the microcapsules according to the invention can also be proofed by stability/storage tests. Even harsh conditions like a standard softener composition and a temperature of 45 °C don't destroy the microcapsules according to the invention.

The present invention relates to microcapsules comprising a shell material obtained by a coacervation process and a core material comprising at least one hydrophobic ingredient. Preferably the shell material comprises a coacervate of at least two polyelectrolytes, preferably gum arabic and chitosan. More preferred the core material comprises one or more further microcapsule/s (template microcapsule/s) comprising a shell material (inner shell material) and a core material which comprises at least one hydrophobic ingredient.

In the most preferred embodiment, the microcapsules according to the invention comprise a shell material and a core material, wherein the shell material is a coacervate of gum arabic and chitosan, cross-linked with glutaraldehyde and the core material comprises at least one hydrophobic ingredient.

The present invention also relates to the use of microcapsules obtained or obtainable by the invention or to composition and products comprising them. Such compositions or products may be either solid or liquid product. According to a particular embodiment, liquid products are preferred. Preferred products and composition are used in the "home- or personal- care" industry, e.g. laundry or cleansing compositions.

The invention will now be further described by way of examples. It will be appreciated that the invention as claimed is not intended to be limited in any way by these examples.

## PARTICULARLY PREFERRED EMBODIMENTS OF THE INVENTION

1. A method is disclosed for producing microcapsules comprising core material and shell material comprising the following steps,

   a) providing a composition comprising at least two solubilized polyelectrolytes,
   b) dadding a core material to the composition of step a) and
   c) initiating the coacervation by changing the pH value into the pH range wherein coacervation of the polyelectrolytes occurs.

2. The method according to embodiment 1, **characterised in that** in step c) the coacervation is initiated by in- or decreasing the pH value into the pH range wherein coacervation of the polyelectrolytes occurs with the provisio that for the combination of gum arabic and chitosan as polyelectrolytes a pH decrease is excluded.

3. The method according to any one of the preceding embodiments, **characterised in that** in step c) the coacervation is initiated by increasing the pH value into the pH range wherein coacervation of the polyelectrolytes occurs.

4. The method according to embodiment 1 or 2, **characterised in that** the composition of step a) has a pH value which is below (<) or above (>) the pH range of the coacervation of the used polyelectrolytes with the provisio that a pH value above the pH range of the coacervation for a composition comprising a combination of gum arabic and chitosan as polyelectrolytes is excluded.

5. The method according to any one of the preceding embodiments, **characterised in that** the composition of step a) has a pH value which is below (<) the pH range of the coacervation of the used polyelectrolytes.

6. The method according to embodiment 5, **characterised in that** the composition of step a) has a pH value, that is greater than or equal to ($\geq$) the p$K_a$ value of one of the polyelectrolytes and below (<) the pH range of coacervation of the used polyelectrolytes.

7. The method according to any one of the preceding embodiments, **characterised in that** in a further step d) the coacervate from step c) is crosslinked.

8. The method according to any one of the preceding embodiments, **characterised in that** at least one cationic polyelectrolyte and one anionic polyelectrolyte is used.

9. The method according to any one of the preceding embodiments, **characterised in that** the polyelectrolyte is selected from the group consisting of gum arabic, sodium carboxymethyl guar gum, plant gums, chitosan, gelatin, casein, pectin, plant proteins, egg white protein, silk protein, alginate, chitin, glycogen, cellulose (in particular carboxymethyl cellulose), starch (e.g. amylose or amylopectin), hyaluronic acid, poly(styrene sulfonate), lignin sulfonate or xanthan gum or mixtures thereof, preferably gum arabic and chitosan or gum arabic and gelatin or gum arabic and soy protein isolate or casein and pectin, more preferred gum arabic and chitosan.

**10.** The method according to any one of the preceding embodiments, **characterised in that** the core material comprises at least one hydrophobic ingredient, preferably consisting of at least one hydrophobic ingredient, or comprises one or more further microcapsule/s (template microcapsule/s) comprising a shell material (inner shell material) and core material which comprises at least one hydrophobic ingredient, preferably consisting of at least one hydrophobic ingredient.

**11.** The method according to embodiment 10, **characterised in that** the hydrophobic ingredient is selected from the group consisting of alcohols, perfume oils, care substances, natural and/or synthetic oils, silicone oils, pesticides, biocides, pigments, phase change materials (PCM), fertilizer, adhesives, insecticides, solvents, lubricants, dyes and cooling substances or mixtures thereof.

**12.** The method according to any one of the preceding embodiments, **characterised in that** the crosslinking step d) is done by adding covalent crosslinker such as an aldehydic compounds, e.g. glyoxal, formaldehyde, glutaraldehyde, genipin or aglycone geniposidic acid, vanillin, ethylene glycol diglycidyl ether (EGDE), hexamethylene-1,6-di-(aminocarboxysulfonate) or mixtures thereof or by adding ionic crosslinkers such as tannic acid or tripolyphosphates, e.g. sodium tripolyphosphate (NaTPP), citric acid or mixtures thereof.

**13.** The method according to any one of the embodiments 10 to 12, **characterised in that** the inner shell material comprises a resin.

**14.** The method according to embodiment 13, **characterised in that** the resin can be obtained by the condensation of thermosetting resins and a subsequent formation of microcapsules using aldehydes, preferably formaldehyde and optionally, wherein the shell material of the microcapsule has been treated with an oxidizing agent preferred with $H_2O_2$ prior to curing of the shell material further optionally supplemented by silica or a silica derivative.

**15.** The method according to embodiment 13, **characterised in that** the resin can be obtained by reacting

i) at least one aromatic alcohol or ethers or esters thereof and
ii) at least one aldehyde component that comprises at least two carbon atoms per molecule, and
iii) in the presence of at least one homopolymer or copolymer of 2-acrylamido-2-methyl-propane sulfonic acid or salts thereof and/or biopolymers such as gum arabic.

**16.** The method according to embodiment 15, **characterised in that** the aromatic alcohol of a) is selected from phenol, cresol (o-, m- and p-cresol), naphthol ($\alpha$- and $\beta$-naphthol), thymol, pyrocatechol,resorcinol, hydroquinone and 1,4-naphthohydroquinone, phloroglucinol, pyrogallol, or hydroxyhydroquinone.

**17.** The method according to embodiment 15, **characterised in that** the aldehydic component b) is selected from valeraldehyde, capronaldehyde, caprylaldehyde, decanal, succindialdehyde, cyclohexanecarboxaldehyde, cyclopentanecarboxaldehyde, 2-methyl-1-propanal, 2-methylpropionaldehyde, acetaldehyde, acrolein, aldosterone, antimycin A, 8'-apo-$\beta$-carotene-8'-al, benzaldehyde, butanal, chloral, citral, citronellal, crotonaldehyde, dimethylaminobenzaldehyde, folinic acid, fosmidomycin, furfural, glutaraldehyde, glyceraldehyde, glycolaldehyde, glyoxal, glyoxylic acid, heptanal, 2-hydroxybenzaldehyde, 3-hydroxybutanal, hydroxymethylfurfural, 4-hydroxynonenal, isobutanal, isobutyraldehyde, methacrolein, 2-methylundecanal, mucochloric acid, N-methylformamide, 2-nitrobenzaldehyde, nonanal, octanal, oleocanthal, orlistat, pentanal, phenylethanal, phycocyanin, piperonal, propanal, propenal, protocatechuic aldehyde, retinal, salicylaldehyde, secologanin, streptomycin, strophanthidin, tylosin, vanillin, or cinnamaldehyde.

**18.** The method according to any one of the preceding embodiments 15 to 17, **characterised in that** the molar ratio of the at least one aromatic alcohol a) to the at least one aldehydic component b), which comprises at least two carbon atoms per molecule, is between 1:2 and 1:3.5, preferably between 1:2.4 and 1:2.8, and is particularly preferably 1:2.6.

**19.** The method according to any one of the preceding embodiments 13 to 18, **characterised in that** a surfactant is used.

**20.** The method according to embodiment 19, **characterised in that** the surfactant is selected from the group consisting of sulfonated lignin, natural dispersants like quillaja saponin, lecithins, gum arabic, pectin, carrageenan, chitosan, chondroitin sulfate, cellulose gum, physically or chemically modified starch, whey protein, pea protein, egg white protein, silk protein, gelatin of fish, proteins of porcine or bovine origin, ester gum, fatty acids or mixtures thereof, preferably is sulfonated lignin.

21. The method according to any one of the preceding embodiments, **characterised in that** it comprises a further step e) of drying the slurry obtained in step d) to obtain dried microcapsules.

22. Microcapsules obtained by a method according to any one of the embodiments 1 to 21.

23. Microcapsules according to embodiment 22, whereas the microcapsules have a hydrodynamic diameter d(90%) between 15 and 150 μm, preferred between 30 and 120 μm, more preferred between 40 and 100 μm, with a standard deviation of ± 30 μm.

24. Microcapsules according to embodiment 22, whereas the microcapsules are stable in storage after drying for at least 14 days, preferably for at least 28 days, particularly preferably for at least 36 days at storage temperatures from 20 to 45 °C and at a relative air humidity of 30 to 70 %.

25. Microcapsules according to any one of the embodiments 13 to 21 and 22, **characterised in that** the microcapsules have a content of synthetic polymer(s) (CSP) less than or equal to (≤) 0.15, wherein the CSP is the quotient of the amount of synthetic polymer(s) encapsulating an amount of core material.

26. A microcapsule slurry comprising microcapsules according to embodiment 22.

27. Microcapsules comprising a shell material obtained by a coacervation process and a core material comprising at least one hydrophobic ingredient according to embodiment 10 or 11.

28. Microcapsules according to embodiment 27 wherein the shell material comprises a coacervate of at least two polyelectrolytes, preferably gum arabic and chitosan.

29. Microcapsules according to embodiment 28, **characterised in that** the core material comprises one or more further microcapsule/s (template microcapsule/s) comprising a shell material (inner shell material) according to any one of the embodiments 13 to 18 and a core material which comprises at least one hydrophobic ingredient according to any one of the embodiments 10 or 11.

30. Compositions comprising microcapsules according to embodiment 22 or embodiment 27.

31. Product comprising microcapsules according to embodiment 22 or embodiment 27 in liquid or powder form.

## EXAMPLES

Unless otherwise indicated or described, all mentioned sizes or size distributions of the microcapsules refer to the measuring method using Dynamic Light Scattering (DLS). The measurements were performed in deionized water (refractive index: 1.333) at room temperature (25 °C) with a RETSCH® Partica Laser Scattering Particle Size Distribution Analyzer LA-950V2. The pictures of the micro capsules were taken with the microscope NIKON Eclipse LV100 equipped for UV-microscopy. For TGA measurements (thermal analysis) a PerkinElmer® Thermogravimetic Analyzer T8000 was used.

### Example 1a - Microcapsules chitosan (Chi) and gum arabic (GA)

The process of microencapsulation of a hydrophobic substance by coacervation of two or more oppositely charged polyelectrolytes according to the invention, based on an emulsion process, can be described as four steps:

Coacervation of chitosan (Chi) and gum arabic (GA)

1. Solution of the coacervation polyelectrolytes:
1 g of chitosan and 2 to 5 g of gum arabic are dissolved in 50 ml of a 1 % solution of acetic acid. The pH of the system is adjusted with 32 % HCl at a pH range slightly above the pK$_a$ of gum arabic. Preferably between a pH of 2.4-2.5 the protonated state of the carboxyl groups of this polyelectrolyte is promoted. The temperature of the preparation is kept at 25 °C until crosslinking.

2. Emulsification of the hydrophobic substance:

After 30 minutes the hydrophobic substance to be coated is added to the polyelectrolyte solution in liquid form and emulsified to the desired microcapsule size by means of a stirrer without additional emulsifiers. For capsule sizes d(90%) < 50 μm chitosan types with a degree of deacetylation $D_{deac}$ about 70 % are preferred.

The core/shell ratio is preferably between 2:3 and 3:2.

After emulsification, 50 ml 1 % acetic acid is added to the emulsion. The aim is to avoid a too high concentration of polyelectrolytes during coacervation. High concentrations cause undesirable agglomeration phenomena during the coating of the oil droplets and/or the disruption of the coacervation phenomena. After the addition of the 1 % acetic acid, the pH value is readjusted to 2.4-2.5 with 32 % HCl if necessary.

3. Coacervation of the oppositely charged polyelectrolytes:

The addition of 20 % NaOH increases the pH value up to the coacervation range of 3.5-4.5. The pH range of coacervation may vary depending on the properties of the polyelectrolytes.

4. Crosslinking of the shell material:

The core material can optionally be further solidified/hardened by cooling down to 5°C. After 30 min at this temperature a dialdehyde is added, preferably glutaraldehyde (2 ml 50 % glutaraldehyde solution). After another 30 min, the batch is heated to 50°C to accelerate the crosslinking reaction. After 2h to 4h at 50°C the batch is cooled down to room temperature. The pH value is then increased to 6.0 with NaOH 20 %.

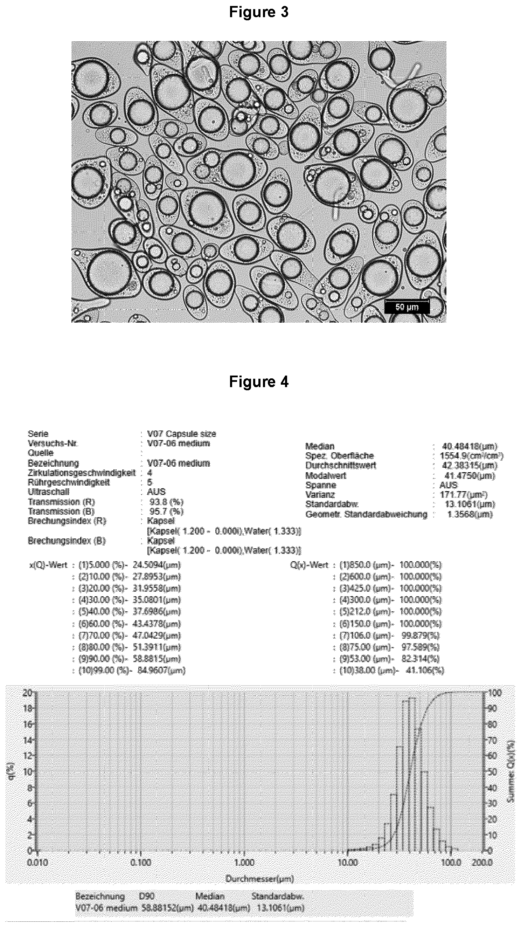

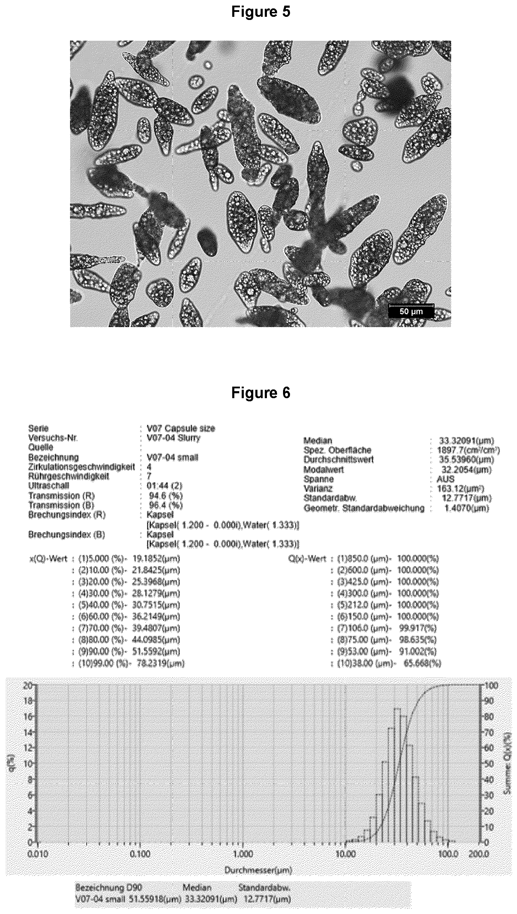

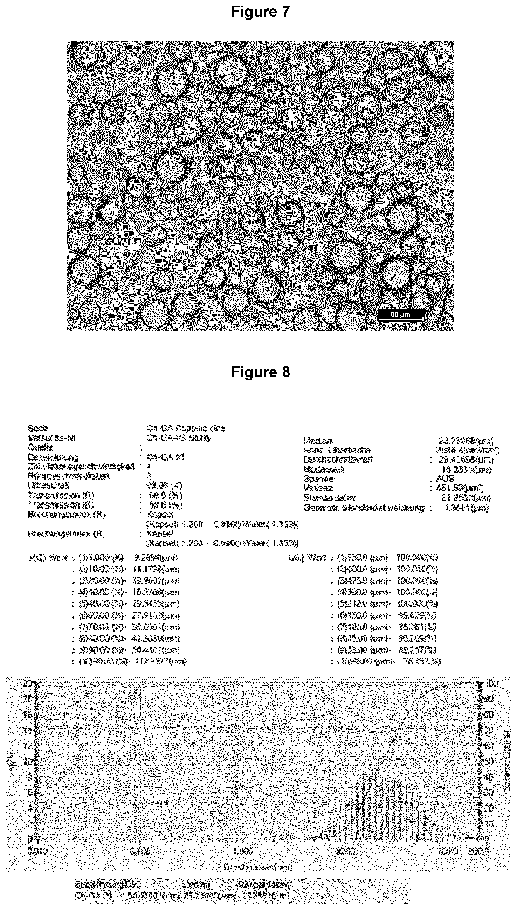

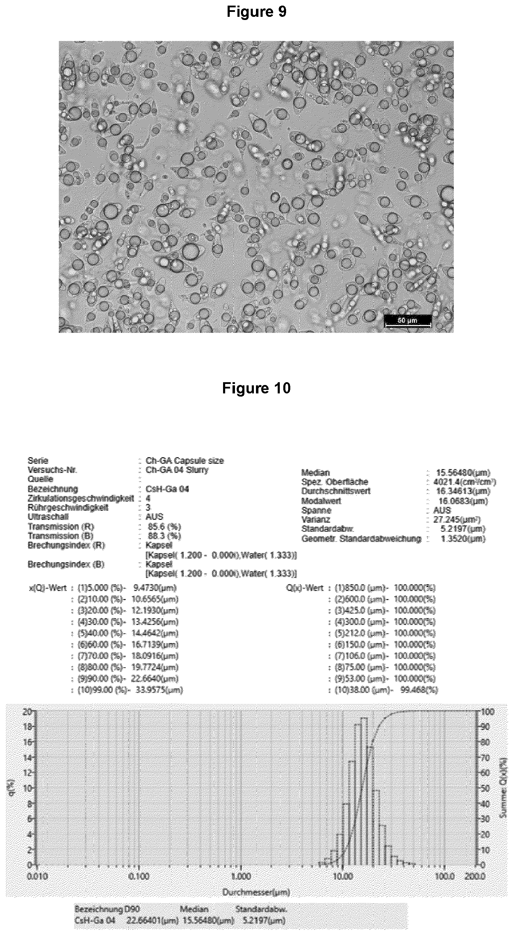

The influence on the microcapsule sizes of stirring speed and the choice of chitosan (different degrees of deacetylation) as well as DLS measurements are shown in Fig. 3 to Fig. 12.

Both the use of other polyelectrolytes (and combinations thereof) and alternative cross-linking agents are included in the disclosure of this invention.

**Example 1b - Microcapsules with different polyelectrolyte combinations**

**Capsules 1 to 4** were produced according to the method described above in Example 1a with ingredients and parameters given in Table 1 a) and 1b). Table 1a) shows the capsule composition and Table 1b) the encapsulation parameters:

**Table 1a** - capsule composition

| | | CAPSULE COMPOSITION | | | | | |
| --- | --- | --- | --- | --- | --- | --- | --- |
| | | SHELL | | | | CORE | |
| | | P1* | P2* | additive | P1:P2 ratio | Protective colloid | substance | Core: Shell rate |
| Capsule 1 | chitosan | gum arabic | - | 1:3 to 1:5 | - | hydrophobic | 1:2 |
| Capsule 2 | gelatin | gum arabic | carboxy-methyl cellulose | 1:1 | - | hydrophobic | 1:2 |
| Capsule 3 | soy protein isolate | gum arabic | hydroxyl-propyl methylcellulose | 1:1 | soy lectine in core | hydrophobic | 1:2 |
| Capsule 4 | casein | pectine | - | 2:1 | - | hydrophobic | 1:2 |
| *P1: polyelectrolyte P1 *P2: polyelectrolyte P2 | | | | | | | |

**Table 1b -** encapsulation parameters

| | | ENCAPSULATION PARAMETERS | | | | | | |
|---|---|---|---|---|---|---|---|---|
| | | INITIAL CONDITIONS | | COACERVATION | | | CROSSLINKING | | |
| | | pH | T [°C] | pH | Acid/base | T [°C] | crosslinker | pH | T [°C] |
| Capsule 1 | | 2.4-2.5 | 25 | 4.0-4.2 | sodium hydroxide | 25 | glutaraldehyde | 4.0-4.5 or 8-9 | 50 |
| Capsule 2 | | 5.5 | 40 | 4.2-4.4 | formic acid | 40 | glutaraldehyde | 9.5-9.8 | 5-10 and 40 |
| Capsule 3 | | 9-10 | 40 | 2.8 | citric acid | 40 | glutaraldehyde/ tannic acid | 2.8-3.0 | 50 |
| Capsule 4 | | 8.5-9.5 | 50 | 3.5 | citric acid | 50 | glutaraldehyde | 3.5 | 50 |

**Example 2 - Multi-layer microcapsules**

The process of microencapsulation of one or more further microcapsule/s (template microcapsule/s) by coacervation of at least two polyelectrolytes according to the invention, based on an emulsion process, can be described as two main steps - the formation of the microcapsules used as core material (template microcapsules) or the simple provision of such template microcapsules followed by the encapsulation with a coacervate.

a) First step - Formation of a first microcapsule (template microcapsule / core of the microcapsule of coacervate)

*Version A: Aminoplastic resin method*

The formation of the microcapsules used as core material (template microcapsule) is based on the process described in patent WO2009/015872 A1. As protective colloids, biodegradable polymers carrying sulfonic acid groups are preferred, especially the sodium, calcium and ammonium salts of lignosulfonic acid.

The microcapsule composition is obtainable by the condensation of thermosetting resins and subsequent formation of microcapsules using aldehydes, preferably formaldehyde. Optionally, the shell material of the capsule can be treated with an oxidizing agent (e.g. $H_2O_2$) prior to curing of the shell material further optionally supplemented by silica or a silica derivative.

*Version B: Formaldehyde-free phenolic resin method*

The formation of the microcapsules used as core material (template microcapsule) is produced according to the process described in patent WO2011/110368 A2.

Those microcapsule's shells comprise a resin which is prepared by reacting a) at least one compound selected from the group consisting of a1) amines and a2) aromatic or heteroaromatic compounds which are unsubstituted or substituted by one or more substituents from the group $C_1$-$C_{20}$ alkyl, OH, OR, COOH, SH, SR, NHCOR, OCOR, halogen, aromatic, wherein R represents a $C_1$-$C_{10}$ alkyl group, with b) at least one aldehydic component containing at least two C atoms per molecule, in the presence of c) at least one copolymer containing units of 2-acrylamido-2-methyl-propanesulfonic acid or its salts (AMPS) and/or 2-acrylamido-2-methyl-propanephosphonic acid or its salts (AMPP) and units of one or more (meth)acrylates. Preferred is a1) selected from the group consisting of urea, melamine and benzoguanamine and a2) is selected from the group consisting of one or more aromatic or heteroaromatic alcohols and/or one or more aromatic or heteroaromatic carboxylic acids, more preferably selected from the group consisting of pyrocatechol, resorcinol, hydroquinone and 1 ,4-naphthohydroquinone, phloroglucinol, pyrogallol, hydroxyhydroquinone, most preferably resorcinol and phloroglucin and the b) the least one aldehydic component is selected from the group consisting of valeraldehyde, caproic aldehyde, caprylic aldehyde, decanal, succindialdehyde, cyclohexane carbaldehyde, cyclopentane carbaldehyde, 2-methyl-1-propanal, 2-methylpropionaldehyde, acetaldehyde, acrolein, aldosterone, antimycin A, 8'-Apo-β-caroten-8'-al, benzaldehyde, butanal, chloral, citral, citronellal, crotonaldehyde, dimethylaminobenzaldehyde, folic acid, fosmidomycin, furfural, glutaraldehyde, glyceraldehyde, glycolaldehyde, glyoxal, glyoxylic acid, Heptanal, 2-hydroxybenzaldehyde, 3-hydroxybutanal, hydroxymethylfurfural, 4-hydroxynonenal, isobutanal, isobutyraldehyde, methacrolein, 2-methylundecanal, mucochloric acid, N-methylformamide, 2-nitrobenzaldehyde, nonanal, octanal, oleocanthal, orlistat, pentanal, phenylethanal, phycocyanin, piperonal, propanal, propenal, protocatechualdehyde, retinal, salicylaldehyde, secologanin, streptomycin, strophanthidin, tylosin, vanillin, cinnamic aldehyde and c) is selected from the group consisting of biopolymers like gum arabic or a terpolymer, which is more preferably composed of units of AMPS or AMPP, preferably AMPS, and one or more (meth)acrylates.

Preferred are template microcapsules according to one of the two mentioned methods from version A or version B. According to the invention, template microcapsules/microcapsules from both methods can also be used as a mixture or independently of each other for the further encapsulation according to the invention's method. In general, all capsules or template microcapsules can be encapsulated by the coacervation method according to the invention.

b) Second step - Encapsulation of the first microcapsule (template microcapsule) by coacervation by a method according to the invention

By coacervation of two oppositely charged polymers (e.g. chitosan and gum arabic) an already encapsulated oil/hydrophobic compound (further microcapsule / template microcapsule) can be coated. Due to the resulting electrostatic forces of attraction, a with reduced water solubility is formed, which deposits in the form of small droplets on the dispersed template microcapsule. Thus, the encapsulation of the template microcapsule occurs.

1. Solution of the coacervation polyelectrolytes:
One part chitosan and four parts gum arabic are dissolved in 56 parts of a 1 % solution acetic acid. The pH of the system is adjusted with 32 % HCl on a pH range slightly above the $pK_a$ of gum arabic. Preferably between a pH of 2.4-2.5 the protonated state of the carboxyl groups of this polyelectrolyte is promoted. The temperature of the preparation is kept at 25°C until crosslinking.

2. Suspending of the first microcapsule / template microcapsule:
After 30 min 17.2 parts of the template microcapsule to be coated are added to the polyelectrolyte solution in slurry form (aqueous suspension) and dispersed by means of a stirrer.
After the addition of the template microcapsule, the pH value is readjusted to 2.4-2.5 with 32 % HCl if necessary.

3. Coacervation of the oppositely charged polyelectrolytes:
The addition of 20 % NaOH increases the pH value up to the coacervation range of 4.0-4.2. The pH range of coacervation may vary depending on the properties of the polyelectrolytes.

4. Crosslinking of the outer shell material:
After 30 minutes, a crosslinking agent, preferably a dialdehyde is added, more preferably glutaraldehyde (1.3 parts). After 15 min, 10 parts of a solution of 10 % gum arabic are added. After another 15 min, the mixture is heated to 50 °C to accelerate the crosslinking reaction. After 2h at 50 °C the batch is cooled down to room temperature. The pH value is then increased to 6.0-6.5 with NaOH 20 %.

Both the use of other polyelectrolytes and alternative cross-linking agents must be included in the disclosure of the invention.

**Example 2a** - **reduction of synthetic resin content in microcapsules**

The calculation of reduction of phenolic polymer content in template microcapsules is based on the coefficient of the amount of polymer (inner shell material) and the amount of core material in the final product (slurry). Commercially available phenolic capsules for laundry are taken as reference.

### Equation 1 – calculation of phenolic polymer reduction

$$Phenolic\ polymer\ reduction\ [\%] = \frac{\dfrac{Phenolic\ polymer_{Ref}}{Core_{Ref}} - \dfrac{Phenolic\ polymer_{Inv}}{Core_{Inv}}}{\dfrac{Phenolic\ polymer_{Ref}}{Core_{Ref}}} \times 100$$

Table 1 shows the reduction of sample R14-03-09 and R14-03-14 in comparison to commercially available capsules wherein the shell materials comprise a phenolic resin (with a CSP of 0.3658).

**Table 2 -** reduction of phenolic resin polymer content in template capsules.

|  | Comercially available phenolic resin capsule for laundry | Sample R14-03-09 | Sample R144-03-14 |
|---|---|---|---|
| Phenolic resin [g] | 77,68 | 1,81 | 3,63 |
| Core [g] | 212,35 | 51,13 | 51,06 |
| CSP | 0,3658 | 0,0355 | 0,0711 |
| Reduction [%] | 0,00 | 90,30 | 80,57 |

The reduction in multilayer microcapsules (R14-03-09) produced by a method according to the invention in comparison to the commercially available capsules is about 90 %; the value for CSP is therefore 0.0355. The reduction in multilayer microcapsules (R14-03-14) produced by a method according to the invention in comparison to the commercially available capsules for laundry is about 80 %; the value for CSP is therefore 0.0711. Both multilayer microcapsules are chitosan capsules with a template microcapsule comprising a thin inner shell material due to reduced phenoplastic resin content.

**Example 3 - Comparison of methods**

In this example two methods, one according to a method of the present invention versus one state of the art method, for the production of microcapsules with a coacervate of chitosan (Chi) and gum arabic (GA) as shell material and Coumarin 1-containing octane as core material are compared.

Both methods (A and B) use the same materials and compositions. Only the starting point of the pH of the composition of the polyelectrolytes differs in both contrasting methods.

A) Method according to the invention - starting point at a pH value which is below (<) the pH range of the coacervation of the used polyelectrolytes:

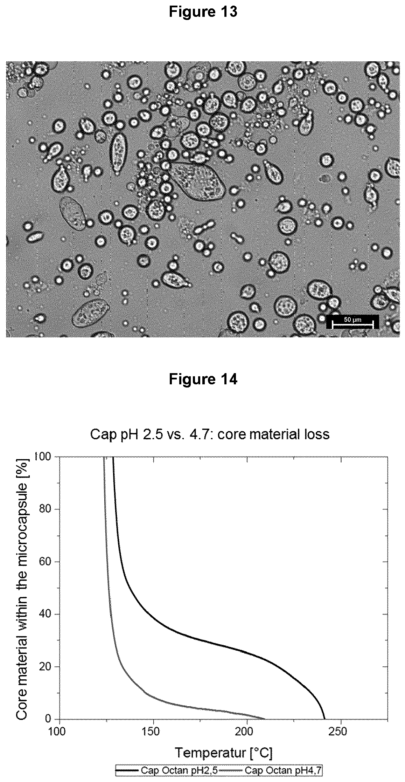

In the first method according to a method of the present invention, an aqueous composition of chitosan and gum arabic (1:4) at pH 2.5 is provided by adding the two polyelectrolytes into water at 25 °C followed by 30 minutes of stirring at 500 rpm (see Fig. 11, A(I)). This composition delivers a solution of the two electrolytes. No phenomes of coacervation are visible. Then the core material Coumarin1-containing octane is added and emulgated at 1300 rpm (see Fig. 11 A(II)). By increasing the pH into the pH range of the coacervation of the used polyelectrolytes (pH = 4.1), the encapsulation of the emulgated droplets of the hydrophobic ingredient by the resulting coacervate occurs (see Fig. 11, A(III)). The subsequent crosslinking hardens the shell material of the microcapsules (see Fig. 11, A(IV) and Fig. 14)

Observations:

- By providing the aqueous composition of chitosan and gum arabic at a pH of 2.5 a clear solution of both polyelectrolytes is resulting (see Fig. 11, A(I)).
- In the emulgation step an effective formation of droplets is achieved (see Fig. 11, A(II)).
- After increasing the pH to 4.1 the coacervate forms a water-insoluble Phase and coats the core material (hydrophobic ingredient). The coating forms complete shell material (see Fig. 11, A(III)).
- By the step of crosslinking the shell material coats densely the core material and is hardened (see Fig. 11, A(IV)). Good and discrete microcapsules are visible. The yield is also correspondingly high (see Fig. 11, A(IV) and Fig.12). Figure 12 shows the resulting slurry under UV light: the bluish color of Coumarin 1 in octane within the microcapsules coated by a dense shell material is clearly visible.

B) Method according to the state of the art - starting point at a pH value which is above (>) the pH range of the coacervation of the used polyelectrolytes:

In the second method according to a method of the state of the art, an aqueous composition of chitosan and gum arabic (1:4) is provided by adding the two polyelectrolytes into water, adjusting the pH 4.7 at 25 °C followed by 30 minutes of stirring at 500 rpm (see Fig. 11, B(I)). Then the core material Coumarin1-containing octane is added and emulgated at 1300 rpm (see Fig. 11 B(II)). By decreasing the pH into the pH range of the coacervation of the used polyelectrolytes (pH = 4.1), the encapsulation of the emulgated droplets of the hydrophobic ingredient by the resulting coacervate occurs (see Fig. 11, B(III)). The subsequent crosslinking hardens the shell material of the microcapsules (see Fig. 11, B(IV) and Fig. 13).

Observations:

- By providing the aqueous composition of chitosan and gum arabic at a pH of 4.7, first phenomes of coacervation are already visible (see Fig. 11, B(I)).
- Due to an incomplete dissolution of polyelectrolytes an effective formation of droplets is not possible (see Fig. 11, B(II)). First coacervate may occur.
- After increasing the pH to 4.1 the coacervate forms a water-insoluble Phase and coats the core material (hydrophobic ingredient). The coating forms complete and densely shell material as well as much incomplete and leaky shell material (see Fig. 11, B(III)).
- During the step of crosslinking a few dense microcapsules are formed. Strong losses of the shell material are clearly visible (see Fig. 11, B(IV)). A deterioration of the capsule stability is to be expected. The product yield is noticeably worse than in comparison to the method according to the present invention (see Fig. 11, B(IV) and Fig. 13). Figure 13 shows the resulting slurry under UV light: less microcapsules and less of the bluish color of Coumarin 1 in octane within the microcapsules coated by the shell material (= less capsule loading).

**Leakage test:**

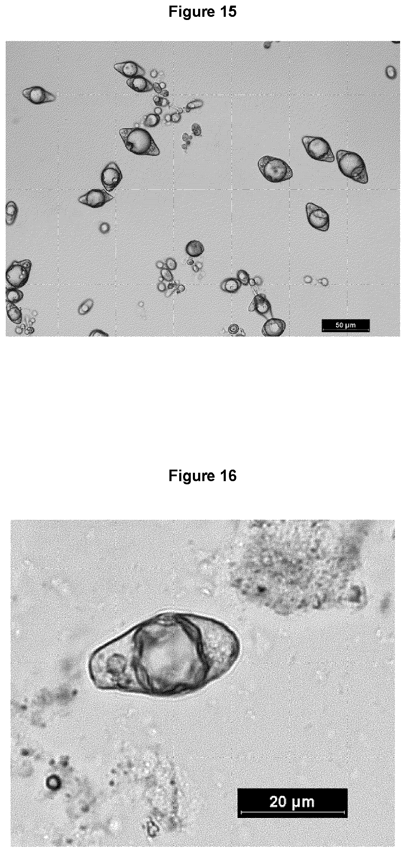

A thermal analysis of the capsules produced was performed. The aim was to determine differences in tightness/leakage. The test temperature program was the following: 1.) Holding for 1.0 min at 30.00 °C, 2.) Heat from 30.00 °C to 90.00 °C at 5.00 °C/min, 3.) Heat from 90.00 °C to 500.00 °C at 10.00 °C/min - Purging gas was nitrogen with a purge gas rate of 20.0 mL/min. Three probes were analyzed: microcapsules according to a method according to the invention A) ("Cap Octan pH2.5"), microcapsules according to a state of the art method B) ("Cap Octan pH4.7") and as a reference the core material octane alone. Considering the theoretical loading of the capsules, core material loss can be represented as a function of temperature: Figure 16 shows the curves of loss of core material for both microcapsules "Cap Octan pH2.5" and "Cap Octan pH4.7" due to the increase of temperature. A deterioration of the microcapsule due to the loss of wall material in the variant with a start pH at 4.7 is clearly visible.

**Example 4 - Capsule resistance in softener**

The aim of this test was to determine the capsule resistance in softener. Therefore two different observation/testing methods were used.

**I. Sensory panel**

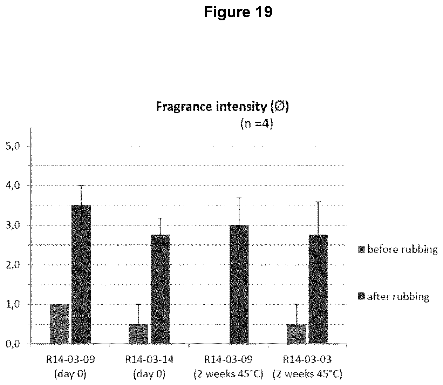

In a sensory panel of four independent test persons multilayer microcapsules according to the invention (chitosan capsules with a template capsule comprising a thin, phenoplastic resin - 80 or 90 % reduced (inner) shell material in comparison with commercially available capsules for laundry) were presented to detected an odour resulting from a leakage of the core material (fragrance oil, coloured with Coumarin 1). In a range from 0: no odour detected... to 5: very strong odour detected the test person rates the olfactory impression of the presented capsules. Two types of multilayer microcapsules according to the invention were tested. Both capsules are chitosan capsules with a template capsule comprising a thin, phenoplastic resin. R14-03-09 has a template capsule which is 90 % reduced in used synthetic polymer and R14-03-14 has a template capsule which is 80 % reduced in used synthetic polymer. Both capsule types were tested at day 0 of storage before and after rubbing as well as after a 14-days storage in a standard softener (purchased from Henkel) at a pH of 3.0 and 45 °C before and after rubbing. Then results are presented in table 2 and figure 19.

Table 3 – results from the sensory panel at day 0 and after 14 days of storage at 45 °C (before and after rubbing) of two different multilayer capsules according to the invention.

| | R14-03-09 (day 0) | | R14-03-14 (day 0) | | R14-03-09 (2 weeks 45°C) | | R14-03-14 (2 weeks 45°C) | |
| --- | --- | --- | --- | --- | --- | --- | --- | --- |
| | before rubbing | after rubbing | before rubbing | after rubbing | before rubbing | after rubbing | before rubbing | after rubbing |
| Ø | 1,0 | 3,5 | 0,5 | 2,8 | 0,0 | 3,0 | 0,5 | 2,8 |
| s | 0,0 | 0,5 | 0,5 | 0,4 | 0,0 | 0,7 | 0,5 | 0,8 |
| Test person 1 | 1 | 4 | 0 | 3 | 0 | 3 | 0 | 3 |
| Test person 2 | 1 | 3 | 0 | 3 | 0 | 3 | 1 | 2 |
| Test person 3 | 1 | 3 | 1 | 2 | 0 | 2 | 0 | 2 |
| Test person 4 | 1 | 4 | 1 | 3 | 0 | 4 | 1 | 4 |

The results show no odour or a very light odour at day 0 before rubbing the capsules. After rubbing them an olfactory boost of fragrance can be detected. No significant performance differences occurs after a 14-days storage at 45 °C in a standard softener composition (pH = 3.0). After rubbing the stored capsules they still show a strong odour even after these harsh conditions.

**II. Microscopy**

For an independent optical determination of these capsules, they were examined under a UV-Microscopy (NIKON Eclipse LV100). Due to the fact, that Coumarin 1 (blueish color) was encapsulated together with the fragrance oil. It was possible to prove the (storage) stability, even in harsh conditions, of the multilayer microcapsules according to the invention. In figure 17 capsules of R14-03-09 and in figure 18 capsules of R14-03-14 are shown after 14 days of storage at 45 °C in a standard softener composition (pH = 3.0). The blueish colour of the encapsulated Coumarin 1 is clearly visible inside intact microcapsules. No leakage can be detected.

**Claims**

1. A method is disclosed for producing microcapsules comprising core material and shell material comprising the following steps,

   a) providing a composition comprising at least two solubilized polyelectrolytes,
   b) adding a core material to the composition of step a) and
   c) initiating the coacervation by changing the pH value into the pH range wherein coacervation of the polyelectrolytes occurs.

2. The method according to claim 1, **characterised in that** in step c) the coacervation is initiated by in- or decreasing the pH value into the pH range wherein coacervation of the polyelectrolytes occurs with the provisio that for the combination of gum arabic and chitosan as polyelectrolytes a pH decrease is excluded.

3. The method according to claim 1 o 2, **characterised in that** in a further step d) the coacervate from step c) is crosslinked.

4. The method according to any one of the preceding claims, **characterised in that** the polyelectrolyte is selected from the group consisting of gum arabic, sodium carboxymethyl guar gum, plant gums, chitosan, gelatin, casein, pectin, plant proteins, egg white protein, silk protein, alginate, chitin, glycogen, cellulose (in particular carboxymethyl cellulose), starch (e.g. amylose or amylopectin), hyaluronic acid, poly(styrene sulfonate), lignin sulfonate or xanthan gum or mixtures thereof, preferably gum arabic and chitosan or gum arabic and gelatin or gum arabic and soy protein isolate or casein and pectin, more preferred gum arabic and chitosan.

5. The method according to any one of the preceding claims, **characterised in that** the core material comprises at least one hydrophobic ingredient, preferably consisting of at least one hydrophobic ingredient, or comprises one or more further microcapsule/s (template microcapsule/s) comprising a shell material (inner shell material) and core material which comprises at least one hydrophobic ingredient, preferably consisting of at least one hydrophobic ingredient.

6. The method according to claim 5, **characterised in that** the hydrophobic ingredient is selected from the group consisting of alcohols, perfume oils, care substances, natural and/or synthetic oils, silicone oils, pesticides, biocides, pigments, phase change materials (PCM), fertilizer, adhesives, insecticides, solvents, lubricants, dyes and cooling substances or mixtures thereof.

7. The method according to any one of the preceding claims, **characterised in that** the crosslinking step d) is done by adding covalent crosslinker such as an aldehydic compounds, e.g. glyoxal, formaldehyde, glutaraldehyde, genipin or aglycone geniposidic acid, vanillin, ethylene glycol diglycidyl ether (EGDE), hexamethylene-1,6-di-(aminocarboxysulfonate) or mixtures thereof or by adding ionic crosslinkers such as tannic acid or tripolyphosphates, e.g. sodium tripolyphosphate (NaTPP), citric acid or mixtures thereof.

8. The method according to any one of the claims 5 to 7, **characterised in that** the inner shell material comprises a resin.

9. The method according to claim 8, **characterised in that** the resin can be obtained by the condensation of thermosetting resins and a subsequent formation of microcapsules using aldehydes, preferably formaldehyde and optionally, wherein the shell material of the microcapsule has been treated with an oxidizing agent preferred with $H_2O_2$ prior to curing of the shell material further optionally supplemented by silica or a silica derivative.

10. The method according to claim 8, **characterised in that** the resin can be obtained by reacting

   i) at least one aromatic alcohol or ethers or esters thereof and
   ii) at least one aldehyde component that comprises at least two carbon atoms per molecule, and
   iii) in the presence of at least one homopolymer or copolymer of 2-acrylamido-2-methyl-propane sulfonic acid or salts thereof and/or biopolymers such as gum arabic.

11. The method according to any one of the preceding claims, **characterised in that** it comprises a further step e) of drying the slurry obtained in step d) to obtain dried microcapsules.

12. Microcapsules obtained by a method according to any one of the claims 1 to 11.

13. Microcapsules according to claim 12, whereas the microcapsules have a hydrodynamic diameter d(90%) between 15 and 150 $\mu$m, preferred between 30 and 120 $\mu$m, more preferred between 40 and 100 $\mu$m, with a standard deviation of $\pm$ 30 $\mu$m.

14. A microcapsule slurry comprising microcapsules according to claim 12.

15. Microcapsules comprising a shell material obtained by a coacervation process and a core material comprising at least one hydrophobic ingredient according to claim 5 or 6.

# Figure 1

# Figure 2

# Figure 3

# Figure 4

| | | | |
|---|---|---|---|
| Serie | : V07 Capsule size | | |
| Versuchs-Nr. | : V07-06 medium | | |
| Quelle | : | Median | : 40.48418(µm) |
| Bezeichnung | : V07-06 medium | Spez. Oberfläche | : 1554.9(cm²/cm³) |
| Zirkulationsgeschwindigkeit | : 4 | Durchschnittswert | : 42.38315(µm) |
| Rührgeschwindigkeit | : 5 | Modalwert | : 41.4750(µm) |
| Ultraschall | : AUS | Spanne | : AUS |
| Transmission (R) | : 93.8 (%) | Varianz | : 171.77(µm²) |
| Transmission (B) | : 95.7 (%) | Standardabw. | : 13.1061(µm) |
| Brechungsindex (R) | : Kapsel | Geometr. Standardabweichung | : 1.3568(µm) |
| | [Kapsel( 1.200 ~ 0.000i),Water( 1.333)] | | |
| Brechungsindex (B) | : Kapsel | | |
| | [Kapsel( 1.200 ~ 0.000i),Water( 1.333)] | | |

x(Q)-Wert : (1)5.000 (%)- 24.5094(µm)
: (2)10.00 (%)- 27.8953(µm)
: (3)20.00 (%)- 31.9558(µm)
: (4)30.00 (%)- 35.0801(µm)
: (5)40.00 (%)- 37.6986(µm)
: (6)60.00 (%)- 43.4378(µm)
: (7)70.00 (%)- 47.0429(µm)
: (8)80.00 (%)- 51.3911(µm)
: (9)90.00 (%)- 58.8815(µm)
: (10)99.00 (%)- 84.9607(µm)

Q(x)-Wert : (1)850.0 (µm)- 100.000(%)
: (2)600.0 (µm)- 100.000(%)
: (3)425.0 (µm)- 100.000(%)
: (4)300.0 (µm)- 100.000(%)
: (5)212.0 (µm)- 100.000(%)
: (6)150.0 (µm)- 100.000(%)
: (7)106.0 (µm)- 99.879(%)
: (8)75.00 (µm)- 97.589(%)
: (9)53.00 (µm)- 82.314(%)
: (10)38.00 (µm)- 41.106(%)

| Bezeichnung | D90 | Median | Standardabw. |
|---|---|---|---|
| V07-06 medium | 58.88152(µm) | 40.48418(µm) | 13.1061(µm) |

## Figure 5

## Figure 6

| Serie | : V07 Capsule size | | |
|---|---|---|---|
| Versuchs-Nr. | : V07-04 Slurry | Median | : 33.32091(µm) |
| Quelle | : | Spez. Oberfläche | : 1897.7(cm²/cm³) |
| Bezeichnung | : V07-04 small | Durchschnittswert | : 35.53960(µm) |
| Zirkulationsgeschwindigkeit | : 4 | Modalwert | : 32.2054(µm) |
| Rührgeschwindigkeit | : 7 | Spanne | : AUS |
| Ultraschall | : 01:44 (2) | Varianz | : 163.12(µm²) |
| Transmission (R) | : 94.6 (%) | Standardabw. | : 12.7717(µm) |
| Transmission (B) | : 96.4 (%) | Geometr. Standardabweichung | : 1.4070(µm) |
| Brechungsindex (R) | : Kapsel | | |
| | [Kapsel( 1.200 - 0.000i),Water( 1.333)] | | |
| Brechungsindex (B) | : Kapsel | | |
| | [Kapsel( 1.200 - 0.000i),Water( 1.333)] | | |

x(Q)-Wert : (1)5.000 (%)- 19.1852(µm)  
: (2)10.00 (%)- 21.8425(µm)  
: (3)20.00 (%)- 25.3968(µm)  
: (4)30.00 (%)- 28.1279(µm)  
: (5)40.00 (%)- 30.7515(µm)  
: (6)60.00 (%)- 36.2149(µm)  
: (7)70.00 (%)- 39.4807(µm)  
: (8)80.00 (%)- 44.0985(µm)  
: (9)90.00 (%)- 51.5592(µm)  
: (10)99.00 (%)- 78.2319(µm)

Q(x)-Wert : (1)850.0 (µm)- 100.000(%)  
: (2)600.0 (µm)- 100.000(%)  
: (3)425.0 (µm)- 100.000(%)  
: (4)300.0 (µm)- 100.000(%)  
: (5)212.0 (µm)- 100.000(%)  
: (6)150.0 (µm)- 100.000(%)  
: (7)106.0 (µm)- 99.917(%)  
: (8)75.00 (µm)- 98.635(%)  
: (9)53.00 (µm)- 91.002(%)  
: (10)38.00 (µm)- 65.668(%)

| Bezeichnung | D90 | Median | Standardabw. |
|---|---|---|---|
| V07-04 small | 51.55918(µm) | 33.32091(µm) | 12.7717(µm) |

## Figure 7

## Figure 8

| | | |
|---|---|---|
| Serie | : | Ch-GA Capsule size |
| Versuchs-Nr. | : | Ch-GA-03 Slurry |
| Quelle | : | |
| Bezeichnung | : | Ch-GA 03 |
| Zirkulationsgeschwindigkeit | : | 4 |
| Rührgeschwindigkeit | : | 3 |
| Ultraschall | : | 09:08 (4) |
| Transmission (R) | : | 68.9 (%) |
| Transmission (B) | : | 68.6 (%) |
| Brechungsindex (R) | : | Kapsel |
| | | [Kapsel( 1.200 - 0.000i),Water( 1.333)] |
| Brechungsindex (B) | : | Kapsel |
| | | [Kapsel( 1.200 - 0.000i),Water( 1.333)] |

| | | |
|---|---|---|
| Median | : | 23.25060(µm) |
| Spez. Oberfläche | : | 2986.3(cm²/cm³) |
| Durchschnittswert | : | 29.42698(µm) |
| Modalwert | : | 16.3331(µm) |
| Spanne | : | AUS |
| Varianz | : | 451.69(µm²) |
| Standardabw. | : | 21.2531(µm) |
| Geometr. Standardabweichung | : | 1.8581(µm) |

x(Q)-Wert : (1)5.000 (%)- 9.2694(µm)
: (2)10.00 (%)- 11.1798(µm)
: (3)20.00 (%)- 13.9602(µm)
: (4)30.00 (%)- 16.5768(µm)
: (5)40.00 (%)- 19.5455(µm)
: (6)60.00 (%)- 27.9182(µm)
: (7)70.00 (%)- 33.6501(µm)
: (8)80.00 (%)- 41.3030(µm)
: (9)90.00 (%)- 54.4801(µm)
: (10)99.00 (%)- 112.3827(µm)

Q(x)-Wert : (1)850.0 (µm)- 100.000(%)
: (2)600.0 (µm)- 100.000(%)
: (3)425.0 (µm)- 100.000(%)
: (4)300.0 (µm)- 100.000(%)
: (5)212.0 (µm)- 100.000(%)
: (6)150.0 (µm)- 99.679(%)
: (7)106.0 (µm)- 98.781(%)
: (8)75.00 (µm)- 96.209(%)
: (9)53.00 (µm)- 89.257(%)
: (10)38.00 (µm)- 76.157(%)

| Bezeichnung | D90 | Median | Standardabw. |
|---|---|---|---|
| Ch-GA 03 | 54.48007(µm) | 23.25060(µm) | 21.2531(µm) |

## Figure 9

## Figure 10

Serie            : Ch-GA Capsule size
Versuchs-Nr.     : Ch-GA 04 Slurry
Quelle          :
Bezeichnung      : CsH-Ga 04
Zirkulationsgeschwindigkeit : 4
Rührgeschwindigkeit  : 3
Ultraschall       : AUS
Transmission (R)   : 85.6 (%)
Transmission (B)   : 88.3 (%)
Brechungsindex (R)  : Kapsel
                [Kapsel( 1.200 - 0.000i),Water( 1.333)]
Brechungsindex (B)  : Kapsel
                [Kapsel( 1.200 - 0.000i),Water( 1.333)]

Median : 15.56480(µm)
Spez. Oberfläche : 4021.4(cm²/cm³)
Durchschnittswert : 16.34613(µm)
Modalwert : 16.0683(µm)
Spanne : AUS
Varianz : 27.245(µm²)
Standardabw. : 5.2197(µm)
Geometr. Standardabweichung : 1.3520(µm)

x(Q)-Wert : (1)5.000 (%)- 9.4730(µm)
: (2)10.00 (%)- 10.6565(µm)
: (3)20.00 (%)- 12.1930(µm)
: (4)30.00 (%)- 13.4256(µm)
: (5)40.00 (%)- 14.4642(µm)
: (6)60.00 (%)- 16.7139(µm)
: (7)70.00 (%)- 18.0916(µm)
: (8)80.00 (%)- 19.7724(µm)
: (9)90.00 (%)- 22.6640(µm)
: (10)99.00 (%)- 33.9575(µm)

Q(x)-Wert : (1)850.0 (µm)- 100.000(%)
: (2)600.0 (µm)- 100.000(%)
: (3)425.0 (µm)- 100.000(%)
: (4)300.0 (µm)- 100.000(%)
: (5)212.0 (µm)- 100.000(%)
: (6)150.0 (µm)- 100.000(%)
: (7)106.0 (µm)- 100.000(%)
: (8)75.00 (µm)- 100.000(%)
: (9)53.00 (µm)- 100.000(%)
: (10)38.00 (µm)- 99.468(%)

Bezeichnung D90     Median     Standardabw.
CsH-Ga 04  22.66401(µm)  15.56480(µm)  5.2197(µm)

## Figure 11

## Figure 12

Figure 13

Figure 14

Cap pH 2.5 vs. 4.7: core material loss

Figure 15

Figure 16

Figure 17

Figure 18

## Figure 19

**Fragrance intensity (Ø)**
**(n =4)**

Europäisches
Patentamt

European
Patent Office

Office européen
des brevets

# EUROPEAN SEARCH REPORT

Application Number

EP 20 18 1096

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2014/044840 A1 (FIRMENICH & CIE [CH]) 27 March 2014 (2014-03-27) * summary of the invention, 1st paragraph; description page 11, 1st paragraph; description page 4, last full paragraph.; claims 1-7, 10; figure 1; example 1 * ----- | 1-15 | INV. B01J13/10 A01N25/28 A61K8/11 A61Q19/00 B01J13/14 B01J13/22 C09B67/02 C11D3/50 F28D20/02 |
| X | EP 1 371 410 A1 (NIZO FOOD RES [NL]) 17 December 2003 (2003-12-17) * example 1 * ----- | 1-15 | |
| X | EP 2 926 894 A1 (GIVAUDAN SA [CH]) 7 October 2015 (2015-10-07) * example 1 * ----- | 1-15 | |

TECHNICAL FIELDS SEARCHED (IPC)

B01J
A61Q
F28F
C09B
C11D
A01N
A61K
F28D

The present search report has been drawn up for all claims

| Place of search | Date of completion of the search | Examiner |
|---|---|---|
| The Hague | 10 November 2020 | Mc Donnell, Shane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 18 1096

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2020

| Patent document cited in search report | | Publication date | Patent family member(s) | | Publication date |
|---|---|---|---|---|---|
| WO 2014044840 | A1 | 27-03-2014 | BR | 112015006469 A2 | 04-07-2017 |
| | | | CN | 104661740 A | 27-05-2015 |
| | | | EP | 2897723 A1 | 29-07-2015 |
| | | | ES | 2753172 T3 | 07-04-2020 |
| | | | JP | 6250055 B2 | 20-12-2017 |
| | | | JP | 2015536811 A | 24-12-2015 |
| | | | US | 2015250689 A1 | 10-09-2015 |
| | | | WO | 2014044840 A1 | 27-03-2014 |
| EP 1371410 | A1 | 17-12-2003 | AU | 2003242713 A1 | 31-12-2003 |
| | | | EP | 1371410 A1 | 17-12-2003 |
| | | | EP | 1551546 A1 | 13-07-2005 |
| | | | WO | 03106014 A1 | 24-12-2003 |
| EP 2926894 | A1 | 07-10-2015 | CN | 106132531 A | 16-11-2016 |
| | | | EP | 2926894 A1 | 07-10-2015 |
| | | | EP | 3126045 A1 | 08-02-2017 |
| | | | JP | 2017512807 A | 25-05-2017 |
| | | | KR | 20160140635 A | 07-12-2016 |
| | | | US | 2017065497 A1 | 09-03-2017 |
| | | | WO | 2015150370 A1 | 08-10-2015 |
| | | | ZA | 201606014 B | 30-08-2017 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018183150 A1
- WO 2009015872 A1
- WO 2011110368 A1
- WO 2010102830 A1
- WO 2011110368 A2